(19) Europäisches Patentamt
European Patent Office
Office européen des brevets

(11) **EP 4 814 043 A1**

(12) **EUROPEAN PATENT APPLICATION**
published in accordance with Art. 153(4) EPC


(43) Date of publication:
**30.09.2026 Bulletin 2026/40**

(21) Application number: **24912111.2**

(22) Date of filing: **21.11.2024**

(51) International Patent Classification (IPC):
***C08J 7/00*** *(2006.01)* ***A61J 1/06*** *(2006.01)*
***A61M 5/315*** *(2006.01)* ***A61M 5/32*** *(2006.01)*

(52) Cooperative Patent Classification (CPC):
**A61J 1/06; A61M 5/315; A61M 5/32; C08J 7/00**

(86) International application number:
**PCT/JP2024/041244**

(87) International publication number:
**WO 2025/142218 (03.07.2025 Gazette 2025/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.12.2023 JP 2023223382**



(71) Applicant: **SUMITOMO RUBBER INDUSTRIES, LTD.**
**Kobe-shi, Hyogo 651-0072 (JP)**

(72) Inventors:
- **KIDA, Yogun**
  **Kobe-shi, Hyogo 651-0072 (JP)**
- **ISHIDA, Hijiri**
  **Kobe-shi, Hyogo 651-0072 (JP)**

(74) Representative: **Manitz Finsterwald Patent- und Rechtsanwaltspartnerschaft mbB**
**Ganghoferstraße 29**
**80339 München (DE)**


(54) **METHOD FOR PRODUCING MEDICAL RUBBER ARTICLE**


(57) [Problem to be solved] An object of the present invention is to provide a method for producing a medical rubber article having a surface with a reduced friction coefficient and tackiness.

[Solution to solve problem] The method for producing the medical rubber article according to the present invention comprises a step of irradiating a cured product of a medical rubber composition containing a halogenated butyl rubber as (a) a base polymer with an ultraviolet ray.


[Fig. 2]

D1
47
40
42
H1
43
41
H2
46
44
H0
H3
45
48
49


EP 4 814 043 A1

## Description

### TECHNICAL FIELD

The present invention relates to a method for producing a medical rubber article, and more specifically relates to a technology for reducing friction coefficient and tackiness of a medical rubber article surface.

### BACKGROUND ART

A halogenated butyl rubber has an excellent gas barrier property and thus is used for a medical rubber article. Since the halogenated butyl rubber has only a small amount of double bonds that can be vulcanized, the surface of the vulcanized rubber article is tacky (has a high tackiness value). Thus, there is a problem that the sliding property is poor, and the rubber articles stick to each other when they are stored for a long period of time. The surface of the vulcanized rubber article is coated with silicone oil or laminated with a fluororesin-based film for a purpose of imparting a high sliding property and reducing frictional resistance.

On the other hand, there is a surface modification method that irradiates a polymer material with an ultraviolet ray to improve properties of the surface of the polymer material.

For example, Patent literature 1 discloses a surface modification method of a material, comprising irradiating a material made from a polymer having a -$CH_2$-bond in a side chain or main chain with an ultraviolet ray having a wavelength ranging from 160 to 310 nm in an inert atmosphere, followed by irradiating the material with an ultraviolet ray having a wavelength of 200 nm or less in an oxidative atmosphere to uniform wettability of the material surface.

Patent literature 2 discloses a surface treatment method of a polymer material substrate, comprising contacting a surface of the polymer material substrate (excluding a fabric substrate) with a vinyl compound represented by a general formula (1) R-CH=$CH_2$ (in the formula, R represents an alkyl group having 6 or more carbon atoms), and irradiating the surface of the polymer material substrate with an ultraviolet ray to impart water repellency to the surface of the polymer material substrate.

### Citation List

### Patent Literature

Patent literature 1: JP H03-128941 A
Patent literature 2: JP 2023-053773 A

### SUMMARY OF THE INVENION

### Technical Problem

The surface of the medical rubber article is coated with the silicone oil or laminated with the fluororesin-based film to improve the sliding property. However, if the medical rubber article coated with the silicone oil contacts with a biological medication, there is a risk that the silicone particle may cause protein aggregation. Thus, the medical rubber article coated with the silicone oil cannot be used for a medical product using a biological medication. Particularly, there is a growing need of silicone oil-free (SOF) for a plunger stopper of a prefilled syringe.

In addition, the laminated medical rubber article laminated with the fluororesin-based film such as a polytetrafluoroethylene (PTFE) film tends to have reduced sealing performance when used as a plunger stopper of a prefilled syringe because the PTFE film has an elastic modulus 100 times higher than a rubber.

A method of reducing the friction coefficient and tackiness of the surface of the medical rubber article without using the silicone oil so as to comply with the SOF regulation is required.

The present invention has been made in view of the above circumstances, and an object of the present invention is to provide a novel method for producing a medical rubber article having a surface with a reduced friction coefficient and reduced tackiness.

### Solution to Problem

The method for producing the medical rubber article according to the present invention comprises a step of irradiating a cured product of a medical rubber composition containing a halogenated butyl rubber as (a) a base polymer

with an ultraviolet ray. The present inventors have found that irradiating a cured product of a medical rubber composition containing a halogenated butyl rubber as (a) a base polymer with an ultraviolet ray greatly modifies the cured product surface such that the cured product surface has a low friction coefficient and low tackiness (tackiness is almost zero), and completed the present invention.

## Effect of the invention

If the method for producing the medical rubber article according to the present invention is used, a medical rubber article having a surface with a reduced friction coefficient and reduced tackiness is obtained.

## BRIEF DESCRIPTION OF THE DRAWINGS

Fig. 1 is an illustrative drawing of one embodiment of the medical rubber article (plunger stopper) according to the present invention,
Fig. 2 is an illustrative drawing of one embodiment of the medical rubber article (plunger stopper) according to the present invention,
Fig. 3 is an illustrative drawing of one embodiment of the medical rubber article (rubber plug) according to the present invention,
Fig. 4 is an illustrative drawing of one embodiment of the medical rubber article (rubber plug) according to the present invention,
Fig. 5 is an illustrative drawing of one embodiment of the medical rubber article (rubber plug for vacuum blood collection tube) according to the present invention,
Fig. 6 is an illustrative drawing of one embodiment of the medical rubber article (rubber plug for vacuum blood collection tube) according to the present invention,
Fig. 7 is an illustrative drawing of one embodiment of the medical rubber article (nozzle cap) according to the present invention, and
Fig. 8 is an illustrative drawing of a measuring method of the friction coefficient of the medical rubber article.

## DESCRIPTION OF EMBODIMENTS

The method for producing the medical rubber article according to the present invention comprises a step of irradiating a cured product of a medical rubber composition containing a halogenated butyl rubber as (a) a base polymer with an ultraviolet ray (hereinafter sometimes simply referred to as "the ultraviolet ray irradiation step").
The cured product of the medical rubber composition used in the production method according to the present invention is obtained by vulcanizing a medical rubber composition containing a halogenated butyl rubber as (a) a base polymer. Firstly, the medical rubber composition will be explained.

<Medical rubber composition>

[(a) Base polymer]

(a) The base polymer contains a halogenated butyl rubber. A crosslinking reaction of the halogenated butyl rubber occurs in the ultraviolet ray irradiation step, which allows (a) the base polymer as a whole to have an increased elastic modulus on the rubber surface and to hardly deform. As a result, the medical rubber article obtained by the production method according to the present invention has a reduced actual contact area on the contact surface with other articles, and thus has a small friction coefficient.
Examples of the halogenated butyl rubber contained in (a) the base polymer include a chlorinated butyl rubber, a brominated butyl rubber, and a bromide of a copolymer of isobutylene and p-methylstyrene. These halogenated butyl rubbers may be used solely, or two or more of them may be used in combination. As the halogenated butyl rubber, the chlorinated butyl rubber or the brominated butyl rubber is preferable. The chlorinated butyl rubber or brominated butyl rubber is, for example, a product obtained by an addition or substation reaction between chlorine or bromine and an isoprene structural moiety (specifically a double bond and/or a carbon atom adjacent to the double bond) of a butyl rubber. It is noted that the butyl rubber is a copolymer obtained by polymerizing isobutylene and a small amount of isoprene. It is noted that the halogenated butyl rubber is a solid at normal temperature (23 °C).
The amount of the halogen in the halogenated butyl rubber is preferably 0.5 mass % or more, more preferably 1 mass % or more, and even more preferably 1.2 mass % or more, and is preferably 5 mass % or less, more preferably 4

mass % or less, and even more preferably 3 mass % or less.

When (a) the base polymer contains the chlorinated butyl rubber or the brominated butyl rubber as the halogenated butyl rubber, the crosslinking reaction occurs at the chlorinated or brominated isoprene moiety in the ultraviolet ray irradiation step.

When (a) the base polymer contains the brominated isobutylene/para-methylstyrene copolymer rubber (BIMS) as the halogenated butyl rubber, the crosslinking reaction occurs at the brominated para-methylstyrene moiety in the ultraviolet ray irradiation step.

Specific examples of the chlorinated butyl rubber include at least one member selected from Exxon (Registered trademark) Chlorobutyl 1066 [halogen amount: 1.25 wt%, Mooney viscosity: 38 $ML_{1+8}$ (125 °C), specific gravity: 0.92] and Exxon Chlorobutyl 5066 [halogen amount: 1.50 wt%, Mooney viscosity: 40 $ML_{1+8}$ (125 °C), specific gravity: 0.92] available from Exxon Mobil Corporation; and LANXESS X_BUTYL (Registered trademark) CB1240 available from LANXESS.

Specific examples of the brominated butyl rubber include at least one member selected from Exxon Bromobutyl 2211 [halogen amount: 2.0 wt%, Mooney viscosity: 32 $ML_{1+8}$ (125 °C), specific gravity: 0.93], Exxon Bromobutyl 2222 [halogen amount: 2.0 wt%, Mooney viscosity: 32 $ML_{1+8}$ (125 °C), specific gravity: 0.93], Exxon Bromobutyl 2235 [halogen amount: 2.1 wt%, Mooney viscosity: 39 $ML_{1+8}$ (125 °C), specific gravity: 0.93], Exxon Bromobutyl 2244 [halogen amount: 2.0 wt%, Mooney viscosity: 46 $ML_{1+8}$ (125 °C), specific gravity: 0.93], Exxon Bromobutyl 2255 [halogen amount: 2.1 wt%, Mooney viscosity: 46 $ML_{1+8}$ (125 °C), specific gravity: 0.93], Exxon Bromobutyl 6222 [halogen amount: 2.4 wt%, Mooney viscosity: 32 $ML_{1+8}$ (125 °C), specific gravity: 0.93], Exxon Bromobutyl 7211 [halogen amount: 2.0 wt%, Mooney viscosity: 32 $ML_{1+8}$ (125 °C), specific gravity: 0.93], and Exxon Bromobutyl 7244 [halogen amount: 2.1 wt%, Mooney viscosity: 46 $ML_{1+8}$ (125 °C), specific gravity: 0.93] available from Exxon Mobil Corporation; and LANXESS X_BUTYL BBX2 available from LANXESS.

(a) The base polymer may contain a rubber component other than the halogenated butyl rubber. Examples of the other rubber components include a butyl rubber, an isoprene rubber, a butadiene rubber, a styrene-butadiene rubber, a natural rubber, a chloroprene rubber, a nitrile rubber such as an acrylonitrile-butadiene rubber, a hydrogenated nitrile rubber, a norbornene rubber, an ethylene-propylene rubber, an ethylene-propylene-diene rubber, an acrylic rubber, an ethylene·acrylate rubber, a fluorine rubber, a chlorosulfonated polyethylene rubber, an epichlorohydrin rubber, a silicone rubber, a urethane rubber, a polysulfide rubber, a phosphazene rubber, and 1,2-polybutadiene. These may be used solely, or two or more of them may be used in combination.

When the other rubber component is used, the amount of the halogenated butyl rubber in (a) the base polymer is preferably 90 mass % or more, more preferably 95 mass % or more, and even more preferably 98 mass % or more. In addition, it is also a preferable embodiment that (a) the base polymer consists of the halogenated butyl rubber.

The medical rubber composition preferably contains (b) a crosslinking agent. (b) The crosslinking agent is blended to crosslink the halogenated butyl rubber component contained in (a) the base polymer. (b) The crosslinking agent is not particularly limited, as long as it is a crosslinking agent capable of crosslinking the halogenated butyl rubber. Examples of (b) the crosslinking agent include sulfur, a metal oxide, a resin crosslinking agent, an organic peroxide, and a triazine derivative. These crosslinking agents may be used solely, or two or more of them may be used in combination.

Examples of the sulfur used as the crosslinking agent include insoluble sulfur, powder sulfur, fine powder sulfur, precipitated sulfur, colloidal sulfur, and sulfur chloride.

Examples of the metal oxide used as the crosslinking agent include magnesium oxide, calcium oxide, zinc oxide, and copper oxide.

Examples of the resin crosslinking agent include alkylphenol formaldehyde resins such as an alkylphenol formaldehyde resin, a thermally reactive phenolic resin, a phenolic dialcohol resin, a bisphenolic resin, and a thermally reactive bromomethylalkylated phenolic resin.

Specific examples of the organic peroxide include dialkyl peroxide, peroxy ester, peroxy ketal, and hydroperoxide. Examples of the dialkyl peroxide include di(2-t-butylperoxyisopropyl)benzene, dicumyl peroxide, 2,5-dimethyl-2,5-di(t-butylperoxy)hexane, t-butylcumyl peroxy, di-t-hexyl peroxy, di-t-butyl peroxy, and 2,5-dimethyl-2,5-di(t-butylperoxy) hexyne-3. Examples of the peroxy ester include t-butylperoxy maleate, t-butylperoxy-3,3,5-trimethyl cyclohexanoate, t-butylperoxy laurate, t-butylperoxyisopropyl monocarbonate, t-hexylperoxy benzoate, 2,5-dimethyl-2,5-di(benzoylperoxy)hexane, t-butylperoxy acetate, and t-butylperoxy benzoate. Examples of the peroxy ketal include 1,1-di(t-hexylperoxy)-3,3,5-trimethylcyclohexane, 1,1-di(t-hexylperoxy)cyclohexane, 1,1-di(t-butylperoxy)-2-methylcyclohexane, 1,1-di(t-butylperoxy)cyclohexane, 2,2-di(t-butylperoxy)butane, n-butyl-4,4-di(t-butylperoxy) valerate, and 2,2-di(4,4-di(t-butylperoxy)cyclohexyl)propane. Examples of the hydroperoxide include p-menthane hydroperoxide, and diisopropylbenzene hydroperoxide. These organic peroxides may be used solely, or two or more of them may be used in combination.

Examples of the triazine derivative used as the crosslinking agent include a compound represented by the general formula (1).

(1)

[In the formula, R is -SH, $-OR^1$, $-SR^2$, $-NHR^3$ or $-NR^4R^5$ ($R^1$, $R^2$, $R^3$, $R^4$ and $R^5$ represent an alkyl group, an alkenyl group, an aryl group, an aralkyl group, an alkylaryl group or a cycloalkyl group. $R^4$ and $R^5$ may be identical to or different from each other.). $M^1$ and $M^2$ are H, Na, Li, K, 1/2Mg, 1/2Ba, 1/2Ca, an aliphatic primary amine, secondary amine or tertiary amine, a quaternary ammonium salt, or a phosphonium salt. $M^1$ and $M^2$ may be identical to or different from each other.]

In the general formula (1), examples of the alkyl group include an alkyl group having 1 to 12 carbon atoms, such as a methyl group, an ethyl group, an n-propyl group, an isopropyl group, an n-butyl group, an isobutyl group, a tert-butyl group, an n-pentyl group, an isopentyl group, a tert-pentyl group, an n-hexyl group, 1,1-dimethylpropyl group, an octyl group, an isooctyl group, 2-ethylhexyl group, a decyl group, and a dodecyl group. Examples of the alkenyl group include an alkenyl group having 1 to 12 carbon atoms, such as a vinyl group, an allyl group, 1-propenyl group, an isopropenyl group, 2-butenyl group, 1,3-butadienyl group, and 2-pentenyl group. The aryl group includes a monocyclic or condensed polycyclic aromatic hydrocarbon group, and examples thereof include an aryl group having 6 to 14 carbon atoms, such as a phenyl group, a naphthyl group, an anthryl group, a phenanthryl group, and an acenaphthylenyl group. Examples of the aralkyl group include an aralkyl group having 7 to 19 carbon atoms, such as a benzyl group, a phenethyl group, diphenylmethyl group, 1-naphthylmethyl group, 2-naphthylmethyl group, 2,2-diphenylethyl group, 3-phenylpropyl group, 4-phenylbutyl group, 5-phenylpentyl group, 2-biphenylmethyl group, 3-biphenylmethyl group, and 4-biphenylmethyl group. Examples of the alkylaryl group include an alkylaryl group having 7 to 19 carbon atoms, such as a tolyl group, a xylyl group, and an octylphenyl group. Examples of the cycloalkyl group include a cycloalkyl group having 3 to 9 carbon atoms, such as a cyclopropyl group, a cyclobutyl group, a cyclopentyl group, a cyclohexyl group, a cycloheptyl group, a cyclooctyl group, and a cyclononyl group.

Specific examples of the triazine derivative represented by the general formula (1) include 2,4,6-trimercapto-s-triazine, 2-methylamino-4,6-dimercapto-s-triazine, 2-(n-butylamino)-4,6-dimercapto-s-triazine, 2-octylamino-4,6-dimercapto-s-triazine, 2-propylamino-4,6-dimercapto-s-triazine, 2-diallylamino-4,6-dimercapto-s-triazine, 2-dimethylamino-4,6-dimercapto-s-triazine, 2-dibutylamino-4,6-dimercapto-s-triazine, 2-di(iso-butylamino)-4,6-dimercapto-s-triazine, 2-dipropylamino-4,6-dimercapto-s-triazine, 2-di(2-ethylhexyl)amino-4,6-dimercapto-s-triazine, 2-dioleylamino-4,6-dimercapto-s-triazine, 2-laurylamino-4,6-dimercapto-s-triazine, 2-anilino-4,6-dimercapto-s-triazine, and their sodium salt or disodium salt.

Among them, 2,4,6-trimercapto-s-triazine, 2-dialkylamino-4,6-dimercapto-s-triazine, or 2-anilino-4,6-dimercapto-s-triazine is preferable, and 2-dibutylamino-4,6-dimercapto-s-triazine is particularly preferable because of easy availability.

In addition, examples of the triazine derivative include one or at least two members selected from 6-[bis(2-ethylhexyl)amino]-1,3,5-triazine-2,4-dithiol, 6-diisobutylamino-1,3,5-triazine-2,4-dithiol, 6-dibutylamino-1,3,5-triazine-2,4-dithiol, 6-dibutylamino-1,3,5-triazine-2,4-dithiol·monosodium, 6-anilino-1,3,5-triazine-2,4-dithiol, and 1,3,5-triazine-2,4,6-trithiol.

In the medical rubber composition used in the present invention, the triazine derivative may be used solely, or two or more of them may be used in combination.

The chlorinated butyl rubber and the brominated butyl rubber have a different crosslinking mechanism from each other, thus it is preferable to select and use a suitable crosslinking component for the crosslinking. When the medical rubber composition contains the chlorinated butyl rubber as the halogenated butyl rubber, the medical rubber composition preferably contains the triazine derivative as (b) the crosslinking agent. In addition, when the medical rubber composition contains the brominated butyl rubber as the halogenated butyl rubber, the medical rubber composition preferably contains the metal oxide as (b) the crosslinking agent.

The amount of (b) the crosslinking agent in the medical rubber composition is preferably 0.2 part by mass or more, more preferably 0.4 part by mass or more, and even more preferably 0.6 part by mass or more, and is preferably 20 parts by mass or less, more preferably 15 parts by mass or less, and even more preferably 10 parts by mass or less, with respect to 100 parts by mass of (a) the base polymer component. This is because if the amount of (b) the crosslinking agent falls within the above range, a rubber having good rubber properties (hardness, tensile strength, Cset) and good processibility (little scorching) can be obtained.

When the chlorinated butyl rubber is used as the halogenated butyl rubber, and the triazine derivative is used as (b) the crosslinking agent, the amount of (b) the crosslinking agent in the medical rubber composition is preferably 0.2 part by mass or more, more preferably 0.4 part by mass or more, and even more preferably 0.6 part by mass or more, and is

preferably 4 parts by mass or less, more preferably 3 parts by mass or less, and even more preferably 2 parts by mass or less, with respect to 100 parts by mass of (a) the base polymer component. This is because if the amount of (b) the crosslinking agent falls within the above range, a rubber having good rubber properties (hardness, tensile strength, Cset) and good processibility (little scorching) can be obtained.

When the brominated butyl rubber is used as the halogenated butyl rubber, and the metal oxide is used as (b) the crosslinking agent, the amount of (b) the crosslinking agent in the medical rubber composition is preferably 1 part by mass or more, more preferably 1.5 parts by mass or more, and even more preferably 2 parts by mass or more, and is preferably 20 parts by mass or less, more preferably 15 parts by mass or less, and even more preferably 10 parts by mass or less, with respect to 100 parts by mass of (a) the base polymer component. This is because if the amount of (b) the crosslinking agent falls within the above range, a rubber having good rubber properties (hardness, tensile strength, Cset) and good processibility (little scorching) can be obtained.

It is preferable that the medical rubber composition does not contain a vulcanization accelerator. This is because the vulcanization accelerator sometimes remains in the final rubber product, and dissolves in the liquid medication kept in a syringe or the like. Examples of the vulcanization accelerator include a guanidine accelerator (e.g. diphenylguanidine), a thiuram accelerator (e.g. tetramethylthiuram disulfide, tetramethylthiuram monosulfide), a dithiocarbamic acid salt accelerator (e.g. zinc dimethyldithiocarbamate), a thiazole accelerator (e.g. 2-mercaptobenzothiazole, dibenzothiazolyl disulfide), and a sulfenamide accelerator (N-cyclohexyl-2-benzothiazole sulfenamide, N-t-butyl-2-benzothiazole sulfenamide).

The medical rubber composition may further contain an acid acceptor. The acid acceptor absorbs chlorine-based gas or bromine-based gas that generates in the crosslinking of the halogenated butyl rubber, thereby functioning to prevent occurrence of crosslinking inhibition or the like caused by these gases. In addition, the acid acceptor functions as an anti-scorching agent in the crosslinking of the halogenated butyl rubber, and also functions to prevent increasement of compression set of the medical rubber article.

Examples of the acid acceptor include hydrotalcite, a metal oxide, and a metal hydroxide.

Examples of the hydrotalcite include a Mg-Al based hydrotalcite, such as $Mg_{4.5}Al_2(OH)_{13}CO_3 \cdot 3.5H_2O$, $Mg_{4.5}Al_2(OH)_{13}CO_3$, $Mg_4Al_2(OH)_{12}CO_3 \cdot 3.5H_2O$, $Mg_6Al_2(OH)_{16}CO_3 \cdot 4H_2O$, $Mg_5Al_2(OH)_{14}CO_3 \cdot 4H_2O$, and $Mg_3Al_2(OH)_{10}CO_3 \cdot 1.7H_2O$. Examples of the metal oxide include magnesium oxide, calcium oxide, and zinc oxide. Examples of the metal hydroxide include calcium hydroxide. These acid acceptors may be used solely, or two or more of them may be used in combination. It is noted that the above-mentioned metal oxide used as the crosslinking agent may also function as the acid acceptor.

The amount of the acid acceptor is preferably 0.5 part by mass or more, more preferably 1 part by mass or more, and is preferably 15 parts by mass or less, more preferably 10 parts by mass or less, with respect to 100 parts by mass of (a) the base polymer component. This is because if the amount of the acid acceptor falls within the above range, occurrence of rust on a mold or the like is suppressed, and a defect that the raw material itself becomes a white speck of foreign matter can be reduced.

The medical rubber composition may further contain a filler. Examples of the filler include an inorganic filler such as clay and talc. Among them, as the filler, the inorganic filler is preferable, and clay or talc is more preferable. The filler functions to adjust the rubber hardness of the medical rubber article, and also functions to lower the production cost of the medical rubber article as a bulking agent.

Examples of the clay include a fired clay and kaolin clay. Specific examples of the clay include SILLITIN (Registered trademark) Z available from HOFFMANN MINERAL LLC, SATINTONE (Registered trademark) W available from ENGELHARD Corporation, N N kaolin clay available from Tsuchiya Kaolin Industry Co., Ltd., and PoleStar200R available from Imerys Specialties Japan Co., Ltd..

Specific examples of the talc include High toron A available from Takehara Kagaku Kogyo Co., Ltd., MICRO ACE (Registered trademark) K-1 available from Nippon Talc Co., Ltd., and Mistron (Registered trademark) Vapor available from Imerys Specialties Japan Co., Ltd..

The amount of the filler in the medical rubber composition is preferably suitably set according to the desired rubber hardness or the like of the medical rubber article. For example, the amount of the filler in the medical rubber composition is preferably 5 parts by mass or more, more preferably 10 parts by mass or more, and even more preferably 20 parts by mass or more, and is preferably 200 parts by mass or less, more preferably 150 parts by mass or less, and even more preferably 100 parts by mass or less, with respect to 100 parts by mass of (a) the base polymer component.

The medical rubber composition may further contain a coloring agent such as titanium oxide and carbon black, a lubricant such as stearic acid, a processing aid, polyethylene glycol functioning as a crosslinking activator, a process oil, or the like in an appropriate proportion.

The medical rubber composition can be obtained by kneading (a) the base polymer, (b) the crosslinking agent, and the other materials which are added where necessary. The kneading can be conducted by using, for example, an open roll, a closed-type kneader, or the like. The kneaded product is preferably molded into a ribbon shape, a sheet shape, a pellet shape or the like, more preferably molded into the sheet shape.

The method for producing the medical rubber article according to the present invention may comprise a step of curing the medical rubber composition. The cured product of the medical rubber composition used in the present invention is obtained by vulcanizing (crosslinking) the medical rubber composition. A cured product of the medical rubber composition in a desired shape is obtained by press molding the kneaded product in the ribbon shape, sheet shape, pellet shape or the like. The crosslinking reaction of the medical rubber composition proceeds when conducting the pressing. For example, the molding temperature is preferably 130 °C or more, more preferably 140 °C or more, and is preferably 200 °C or less, more preferably 190 °C or less. The molding time is preferably 2 minutes or more, more preferably 3 minutes or more, and is preferably 60 minutes or less, more preferably 30 minutes or less. The molding pressure is preferably 0.1 MPa or more, more preferably 0.2 MPa or more, and is preferably 10 MPa or less, more preferably 8 MPa or less.

At least one part of the surface of the cured product of the medical rubber composition to which the present invention is applied may be covered with an inert resin layer. For example, at least one part of the surface of the cured product of the medical rubber composition is covered with the inert resin layer by laminating the inert resin film onto a sheet made from the medical rubber composition, followed by press molding.

The inert resin layer covers at least one part of the surface of the medical rubber article, and is preferably appropriately laminated on at least one part of the surface of the medical rubber article based on the shape of the medical rubber article. Particularly, the surface of the medical rubber article contacting with chemicals is preferably provided with the inert resin layer. In this embodiment, the inert resin layer exhibits good chemical resistance, and the sliding property of the surface having no inert resin layer formed thereon can be improved by the ultraviolet ray irradiation.

The resin constituting the inert resin layer is not particularly limited, and examples thereof include at least one fluororesin selected from the group consisting of tetrafluoroethylene·ethylene copolymer (ETFE), polytetrafluoroethylene (PTFE), polychlorotetrafluoroethylene (PCTFE), and a resin other than the fluororesin, from the viewpoint of obtaining good chemical resistance.

The tetrafluoroethylene·ethylene copolymer (ETFE) is obtained by copolymerizing ethylene and tetrafluoroethylene in a molar ratio ranging from 30/70 to 70/30, and there is also modified ETFE in which other components are further copolymerized for a purpose of modification. Examples of the other components include a fluorine-containing olefin and a hydrocarbon-based olefin. Specifically, the other components include an $\alpha$-olefin such as propylene and butene, a fluorine-containing olefin such as hexafluoropropylene, vinylidene fluoride, perfluorobutyl ethylene and trifluorochloroethylene, a vinyl ether such as ethylene vinyl ether, perfluoromethyl vinyl ether and perfluoropropyl vinyl ether, and a fluorine-containing acrylate, and are copolymerized in an amount of about 2 to 10 mole % to modify ETFE.

As the modified ETFE, ETFE having a functional group imparting adhesion can be preferably used, and examples of the functional group include a carboxyl group, an anhydride group, an epoxy group, a hydroxyl group, an isocyanate group, an ester group, an amide group, an aldehyde group, an amino group, a cyano group, a carbon-carbon double bond, a sulfonic acid group, and an ether group. In addition, examples of the commercial product of the modified ETFE include Fluon AH-2000 available from Asahi Glass Co., Ltd..

Examples of the resin other than the fluororesin include an olefin resin. Examples of the olefin resin include a polyethylene resin such as polyethylene, an ethylene-propylene copolymer, an ethylene-propylene-non-conjugated diene copolymer, an ethylene-butene copolymer, an ethylene-hexene copolymer, an ethylene-octene copolymer, an ethylene-vinyl acetate copolymer, an ethylene-vinyl alcohol copolymer, an ethylene-ethyl acrylate copolymer, and a chlorinated polyethylene; a polypropylene resin such as polypropylene, a propylene-ethylene random copolymer, a propylene-ethylene block copolymer, and a chlorinated polypropylene; polybutene, polyisobutylene, polymethylpentene, and a copolymer of a cyclic olefin, and polyethylene (in particular, an ultrahigh molecular weight polyethylene (UHMWPE)) is preferable. In addition, the olefin resin may contain fluorine.

The thickness of the inert resin film may be suitably adjusted according to the shape or size of the medical rubber article, and is preferably 10 $\mu$m or more, more preferably 20 $\mu$m or more, and even more preferably 30 $\mu$m or more, and is preferably 150 $\mu$m or less, more preferably 130 $\mu$m or less, and even more preferably 110 $\mu$m or less. This is because if the thickness of the inert resin film falls within the above range, tearing of the film does not occur during the molding of the product, and wrinkling or lifting of the film on the surface of the molded product does not occur, thereby achieving a balance between moldability and product characteristics.

The arithmetic mean roughness Ra of the inert resin film ranges from 0.01 to 0.03 $\mu$m for a casting film or an extruding film, and is 0.10 $\mu$m for a skiving film, however, if the surface roughness of the mold is set to 0.03 $\mu$m or less, a medical rubber article having excellent liquid tightness and air tightness can be obtained. The lower limit of Ra of the inert film itself is not particularly limited.

The inert resin film is preferably subjected to a treatment for improving adhesion to the rubber or the like. Examples of the treatment for improving the adhesion include a chemical treatment method, treatment of roughening the surface of the film, and a combination thereof, and specific examples thereof include sodium treatment, glow discharge treatment, plasma treatment (discharge treatment) under atmosphere or vacuum, excimer laser treatment (discharge treatment), and ion beam treatment.

<Ultraviolet ray irradiation step>

The method for producing the medical rubber article according to the present invention comprises a step of irradiating the cured product of the medical rubber composition with an ultraviolet ray (ultraviolet ray irradiation step). The cured product of the medical rubber composition to be irradiated with the ultraviolet ray may have a final shape of the medical rubber product or a preform before the medical rubber composition has not yet been molded into the final shape.

The method of irradiating the cured product of the medical rubber composition with the ultraviolet ray is not particularly limited, and examples thereof include a method of irradiating the surface of the cured product of the medical rubber composition with a light source emitting the ultraviolet ray.

The wavelength of the ultraviolet ray is preferably 160 nm or more, more preferably 165 nm or more, and even more preferably 170 nm or more. This is because if the wavelength of the ultraviolet ray is 160 nm or more, the surface modification becomes possible. In addition, the upper limit of the wavelength of the ultraviolet ray is not particularly limited, and is preferably 380 nm or less, more preferably 300 nm or less, and even more preferably 200 nm or less. If the wavelength of the ultraviolet ray is 380 nm or less, the ultraviolet ray has high energy, and thus the halogenated butyl rubber has high crosslinking efficiency and hardly deforms. In addition, the ultraviolet ray having the high energy causes the low molecular weight component that contributes to the surface tackiness of the cured product of the medical rubber composition to decompose and evaporate, thereby achieving low tackiness. Further, since the evaporation of the low molecular weight component causes the surface of the cured product to become rough, the friction coefficient can be further reduced. Among them, a vacuum ultraviolet ray having a wavelength of 200 nm or less is particularly preferable, because the effect of the present invention can be achieved more effectively.

The light source emitting the ultraviolet ray is not particularly limited, as long as the light source can emit an ultraviolet ray having a wavelength falling with the above range. For example, a low-pressure mercury lamp, a high-pressure mercury lamp, or an excimer lamp can be used. Particularly, the excimer lamp is preferable because the energy is intensive and the surface modification can be conducted in a relatively short time. The excimer lamp emits an ultraviolet ray having a different wavelength depending on the type of discharge gas to be used. For example, an ultraviolet ray having a central wavelength of 172 nm is emitted if xenon ($Xe_2$) is used, an ultraviolet ray having a central wavelength of 308 nm is emitted if xenon chloride (XeCl) is used, an ultraviolet ray having a central wavelength of 283 nm is emitted if xenon bromide (XeBr) is used, an ultraviolet ray having a central wavelength of 253 nm is emitted if xenon iodide (XeI) is used, an ultraviolet ray having a central wavelength of 193 nm is emitted if argon fluoride (ArF) is used, an ultraviolet ray having a central wavelength of 165 nm is emitted if argon bromide (ArBr) is used, an ultraviolet ray having a central wavelength of 222 nm is emitted if krypton chloride (KrCl) is used, and an ultraviolet ray having a central wavelength of 207 nm is emitted if krypton bromide (KrBr) is used, and these ultraviolet rays are also called "excimer UV light". In the present invention, the excimer lamp emitting a vacuum ultraviolet ray having a central wavelength of 200 nm or less is preferable, and the excimer lamp using xenon (central wavelength: 172 nm) is particularly preferable.

In the ultraviolet ray irradiation step, the cumulative illuminance of the ultraviolet ray to the cured product of the medical rubber composition is preferably 1000 mJ/cm$^2$ or more, more preferably 3000 mJ/cm$^2$ or more, and even more preferably 5000 mJ/cm$^2$ or more. If the cumulative illuminance of the ultraviolet ray to the cured product of the medical rubber composition is 1000 mJ/cm$^2$ or more, the halogenated butyl rubber is sufficiently crosslinked, and simultaneously the low molecular weight component contributing to the surface tackiness of the cured product of the medical rubber composition is more easily decomposed. In addition, the upper limit of the cumulative illuminance of the ultraviolet ray to the cured product of the medical rubber composition is not particularly limited, and is preferably 50000 mJ/cm$^2$ or less, more preferably 45000 mJ/cm$^2$ or less, and even more preferably 40000 mJ/cm$^2$ or less. This is because if the cumulative illuminance of the ultraviolet ray to the cured product of the medical rubber composition is 50000 mJ/cm$^2$ or less, a balance between a lifetime of an irradiating device and efficiency of surface modification can be achieved. It is noted that the cumulative illuminance of the ultraviolet ray to the cured product of the medical rubber composition is a total illuminance (arrival illuminance) of the ultraviolet ray arriving at the surface of the cured product of the medical rubber composition, and can be calculated by multiplying the intensity (arrival intensity) of the ultraviolet ray arriving at the surface of the cured product of the medical rubber composition by the irradiating time of the ultraviolet ray.

The arrival intensity and irradiating time of the ultraviolet ray are suitably adjusted such that the cumulative illuminance falls within the above range, and generally, it is preferable that the arrival intensity of the ultraviolet ray is set to 10 mW/cm$^2$/sec to 100 mW/cm$^2$/sec, and the irradiating time of the ultraviolet ray is set to 10 seconds to 5000 seconds. This is because if the arrival intensity and irradiating time of the ultraviolet ray are set in these ranges, the cumulative illuminance easily falls within the above range.

The distance between the surface of the cured product of the medical rubber composition and the light source (lamp) emitting the ultraviolet ray is not particularly limited, and is preferably set to 1 mm to 20 mm from the viewpoint of increasing the uniformity of the ultraviolet ray irradiation.

In the present invention, the surface of the cured product can be greatly modified such that the surface of the cured product has a low friction coefficient and low tackiness by irradiating the cured product of the medical rubber

composition with the ultraviolet ray.

In the production method according to the present invention, when at least one part of the surface of the cured product of the medical rubber composition is covered with the inert resin layer, it is preferable to irradiate the exposed rubber surface that is not covered with the inert resin layer with the ultraviolet ray. Only one part of the exposed rubber surface may be irradiated with the ultraviolet ray, or alternatively, the whole exposed rubber surface may be irradiated with the ultraviolet ray.

The method for producing the medical rubber article according to the present invention may comprise a step of processing the cured product of the medical rubber composition into a predetermined shape, a step of washing, a step of sterilizing, and a step of drying. For example, the medical rubber article is produced by irradiating the cured product of the medical composition with the ultraviolet ray, cutting and removing unnecessary portions thereof, processing the cured product into a predetermined shape, and further washing, sterilizing, drying and packing the cured product. It is noted that cutting and removing the unnecessary portions and processing the cured product into the predetermined shape may be conducted before irradiating the cured product of the medical rubber composition with the ultraviolet ray.

The medical rubber article obtained by the production method according to the present invention can achieve a low friction coefficient and low tackiness, even if silicone oil is not used. Accordingly, the medical rubber article according to the present invention can be appropriately used as a medical rubber article that is required to have a low friction coefficient and low tackiness, while complying with the SOF regulation.

It is preferable that the medical rubber article obtained by the production method according to the present invention is not coated with silicone oil from the viewpoint of complying with the SOF regulation. Examples of the silicone oil include dimethyl polysiloxane, methylphenyl polysiloxane and their modified products.

Examples of the medical rubber article obtained by the production method according to the present invention include sliding or sealing articles such as a rubber plug or sealing member for a container of various pharmaceutic formulations of a liquid formulation, a powder formulation and a lyophilized formulation, a rubber plug for a vacuum blood collection tube, a plunger stopper (gasket) for a prefilled syringe, and a nozzle cap. Among them, the medical rubber article that is required to have a low friction coefficient or low tackiness (e.g. the rubber plug or the plunger stopper) is preferable, and the plunger stopper that is required to have an excellent sliding property is particularly preferable.

Among them, the rubber hardness of the rubber plug or sealing member for the vial, infusion preparation container or the like, expressed as Durometer Type A hardness (Shore A hardness) measured in accordance with the measuring method specified in Japanese Industrial Standard JIS K6253-3: 2012 "Rubber, vulcanized or thermoplastic-Determination of hardness-Part 3: Durometer method", is preferably 35 or more and is preferably 60 or less.

In addition, the above-mentioned Shore A hardness of the sliding or sealing member such as the gasket for the prefilled syringe and the nozzle cap is preferably 40 or more and is preferably 70 or less.

The rubber hardness of the medical rubber article may be adjusted by varying the mixing ratio of materials.

Next, specific examples of the medical rubber article to which the present invention is applied will be explained.

<Plunger stopper>

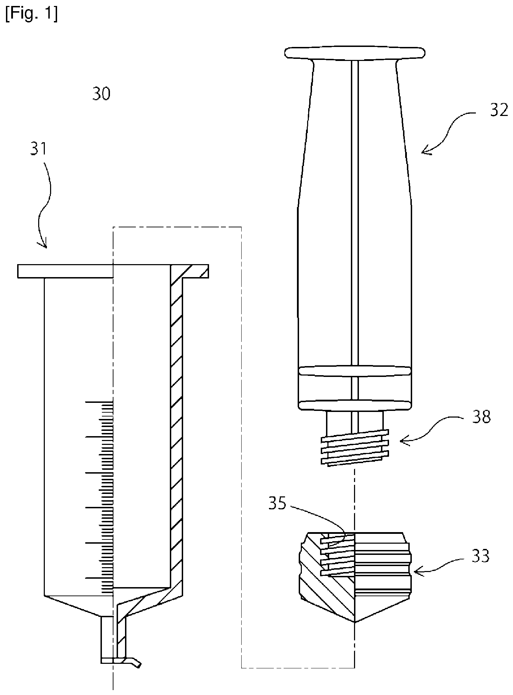

Fig. 1 is a figure showing a medical syringe (a syringe called prefillable syringe 30) in a disassembled state in which the medical rubber article according to the present invention is used. In Fig. 1, a syringe barrel 31 and a plunger stopper 33 are shown in half section. The prefillable syringe 30 comprises a cylindrical syringe barrel 31, a plunger 32 that is coupled to the syringe barrel 31 and is capable of reciprocating in the syringe barrel 31, and a plunger stopper 33 attached to the tip of the plunger 32.

The plunger 32 is constituted, for example, by resin plates having a cross-section shaped like a cross, and the tip thereof is provided with a head 38 to which the plunger stopper 33 is attached. The head 38 is formed integrally with the plunger 32, made from a resin, and processed into a male threaded shape. The plunger stopper 33 has a roughly cylindrical shape with a short axis, and the tip surface thereof has, for example, a mountain-shaped with an obtuse angle and protruding along the axial center. Further, a mating recess 35 that has a female threaded shape and is carved axially from the rear end face is formed. The plunger stopper 33 is attached to the tip of the plunger 32 by screwing the head 38 of the plunger 32 into the mating recess 35 of the plunger stopper 33.

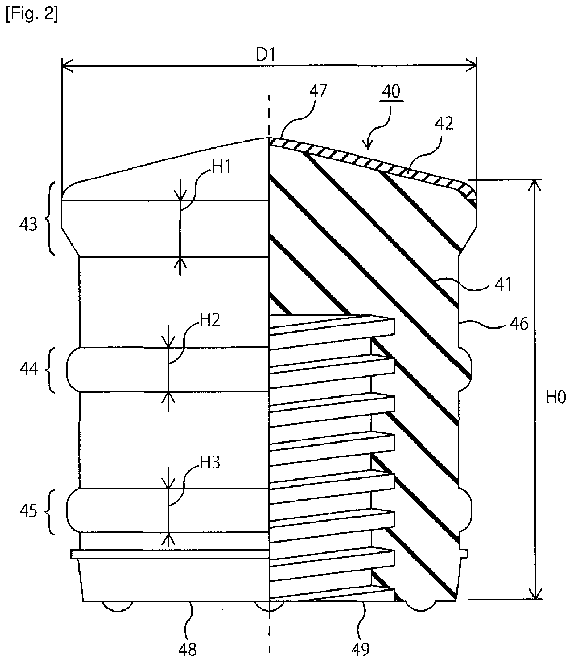

Fig. 2 is a front view of an example of a plunger stopper in half-section. The plunger stopper 40 comprises a main body 41 composed of the cured product of the medical rubber composition, and an inert resin layer 42 covering one part of the surface of the main body. The plunger stopper 40 has a liquid-contacting surface 47 facing a liquid medication and a sliding surface 46 contacting with a syringe barrel.

In the embodiment shown in Fig. 2, when the plunger stopper 40 is inserted into the syringe barrel, only the mountain-shaped liquid-contacting surface 47 that contacts with the liquid medication is covered with an inert resin layer 42. As the inert resin layer 42, for example, a polytetrafluoroethylene film is preferable.

The sliding surface (outer circumferential surface) 46 of the plunger stopper 40 that contacts with the syringe barrel is not provided with the inert resin layer 42 and is irradiated with the ultraviolet ray. The sliding surface (outer

circumferential surface) 46 has a high sliding property.

The plunger stopper 40 has a short cylindrical shape, and has a plurality of annular ribs 43, 44, 45 on the cylindrical outer circumferential surface 46. The annular ribs slidingly contact with the inner circumferential surface of the syringe barrel. The plurality of annular ribs are arranged axially from the tip end face (liquid-contacting surface) 47 toward the rear end face 48 of the plunger stopper. The number of the annular ribs is preferably, but not particularly limited to,1 or more, and is more preferably 2 or more, most preferably 3 or more, and is preferably 6 or less, more preferably 5 or less, and even more preferably 4 or less.

The plunger stopper 40 in Fig. 2 has a first annular rib 43, a second annular rib 44 and a third annular rib 45 starting from the tip side. The compression ratio in the radial direction of the first annular rib 43 on the tip side is preferably 1% or more, more preferably 2% or more, and even more preferably 3% or more, and is preferably 10% or less, more preferably 9% or less, and even more preferably 8% or less. The compression ratio is calculated from an outer diameter D1 of the uncompressed annular rib and an inner diameter R of the syringe barrel, based on the following formula.

$$\text{Compression ratio (\%)} = 100\times(D1-R)/D1$$

The linear length H1 (axial length) of the sliding contact portion of the annular rib 43 on the tip side relative to the linear length Ho (axial length) of the cylindrical outer circumferential surface is preferably 1% or more, more preferably 3% or more, and even more preferably 6% or more, and is preferably 25% or less, more preferably 20% or less, and even more preferably 15% or less.

Each of the linear length H2 (axial length) of the sliding contact portion of the second annular rib 44 and the linear length H3 (axial length) of the sliding contact portion of the third annular rib 45 relative to the linear length Ho (axial length) of the cylindrical outer circumferential surface is preferably 1% or more, more preferably 2% or more, and even more preferably 3% or more, and is preferably 15% or less, more preferably 14% or less, and even more preferably 13% or less, respectively.

It is noted that the plunger stopper is sometimes referred to as a stopper or gasket.

<Rubber plug>

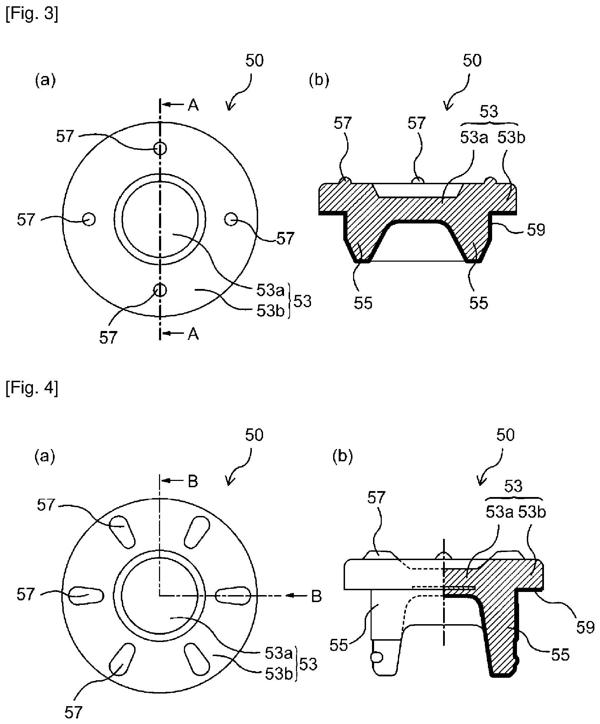

Fig. 3 is an illustrative drawing for illustrating an example of a medical plug (more specifically, a rubber plug for a vial) to which the present invention is applied. Fig. 3 (a) is a plan view, and Fig. 3 (b) is a cross-sectional view along a line A-A in Fig. 3 (a).

The medical plug 50 has a top plate 53 and a cylindrical leg 55 extending downward from the lower surface of the top plate 53. The top plate 53 and the leg 55 are composed of the cured product of the rubber composition. The leg 55 fits into the mouth of a medical container when the medical container is plugged with the medical plug according to the present invention. In Fig. 3 (b), the opposite inner surfaces of the cylindrical legs 55 are tapered so that the distance between the inner surfaces of the legs gradually decreases from the bottom to the top (the top surface side).

The top plate 53 is circular in a plan view. The top plate 53 has a puncture portion 53a that allows an injection needle of a syringe to penetrate, and a flange portion 53b that contacts with the upper edge face of the mouth of the medical container when the medical container is plugged.

The top surface side of the flange portion 53b is provided with a protrusion 57 for preventing the medical plug 50 from sticking to other rubber plug.

The puncture portion 53a is an area for inserting an injection needle to draw the liquid medication inside the container in the top plate 53. The puncture portion 53a is circular in a plan view and is located in the center of the top plate 53. In addition, the puncture portion 53a is formed as a recess from the top surface.

In the embodiment of Fig. 3, the lower surface of the top plate 53 and the surface of the leg 55 are wholly covered with an inert resin layer 59. It is noted that the lower surface of the top plate 53 and the surface of the leg 55 may be partially coated with the inert resin layer 59. As the inert resin layer 59, a polytetrafluoroethylene film is preferable.

The upper surface of the top plate 53 is not provided with an inert resin layer, and is irradiated with the ultraviolet ray. Since a friction coefficient and tackiness of the upper surface of the top plate 53 is reduced, the problem that the rubber plugs stick to each other is improved.

Fig. 4 is an illustrative drawing showing another embodiment of a medical rubber plug 50 to which the present invention is applied. Fig. 4 (a) is a plan view, and Fig. 4 (b) is a cross-sectional view along a line B-B in Fig. 4 (a). In the medical rubber plug 50 in Fig. 4, the explanation for the parts that share the same configuration as those in Fig. 3 is omitted.

The medical rubber plug 50 in this embodiment has bifurcated legs 55 extending from the lower surface of the top plate 53. In Fig. 4 (b), the opposite inner surfaces of the bifurcated legs 55 are tapered so that the distance between the inner surfaces of the legs gradually decreases from the bottom to the top (the top surface side). In the embodiment of Fig. 4, the top plate 53 and the leg 55 are composed of the cured product of the rubber composition, and the lower surface of the

top plate 53 and the surface of the leg 55 are wholly covered with an inert resin layer 59. It is noted that the lower surface of the top plate 53 and the surface of the leg 55 may be partially covered with the inert resin layer 59.

The upper surface of the top plate 53 is not provided with an inert resin layer, and is irradiated with the ultraviolet ray. Since a friction coefficient and tackiness of the upper surface of the top plate 53 is reduced, the problem that the rubber plugs stick to each other is improved.

The top surface of the top plate 53 of the medical rubber plug 50 in Fig. 3 and Fig. 4 may be provided with a nylon film layer. If the top surface of the medical rubber plug 50 is provided with the nylon film layer, mechanical transportability during the production of pharmaceuticals can be ensured. In addition, if the top surface of the medical rubber plug 50 is provided with the nylon film layer, surface slipperiness of the top surface can be increased, and occurrence of needle fragments during the insertion of the injection needle can be prevented.

<Rubber plug for vacuum blood collection tube>

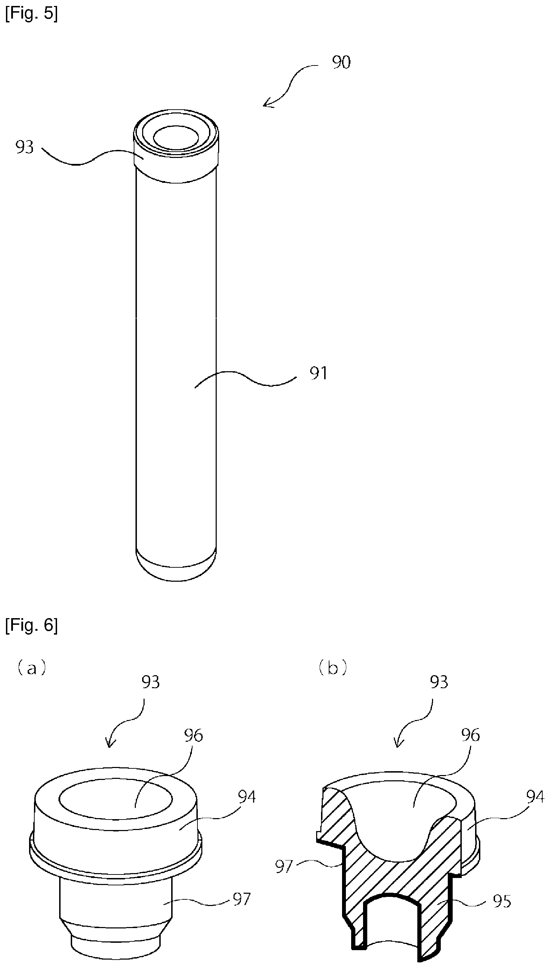

Fig. 5 is an illustrative drawing showing an example of a vacuum blood collection tube. The vacuum blood collection tube 90 is composed of a closed-end tube 91 and a rubber plug 93 sealing the opening of the closed-end tube 91, and is designed to automatically collect blood by reducing the pressure inside the blood collection tube.

Fig. 6 is an illustrative drawing for illustrating an example of a medical plug to which the present invention is applied, i.e. an illustrative drawing showing an example of a rubber plug for a vacuum blood collection tube. Fig. 6 (a) is a perspective view, and Fig. 6 (b) is a cross-sectional view. The rubber plug for the vacuum blood collection tube has a top plate 94 and a cylindrical leg 95 extending downward from the lower surface of the top plate 94. The top plate 94 and the leg 95 are composed of an elastomer. The leg 95 fits into the mouth of the vacuum blood collection tube when the vacuum blood collection tube is plugged with the rubber plug. The center of the top plate 94 is provided with a puncture portion 96 that is an area for inserting an injection needle. The puncture portion 96 is recessed from the top surface. The lower surface of the top plate 94 and the surface of the leg 95 are wholly covered with an inert resin layer 97. It is noted that the lower surface of the top plate 94 and the surface of the leg 95 may be partially covered with the inert resin layer 97.

The upper surface of the top plate 94 is not provided with an inert resin layer, and is irradiated with the ultraviolet ray. Since a friction coefficient and tackiness of the upper surface of the top plate 94 is reduced, the problem that the rubber plugs stick to each other is improved.

<Nozzle cap>

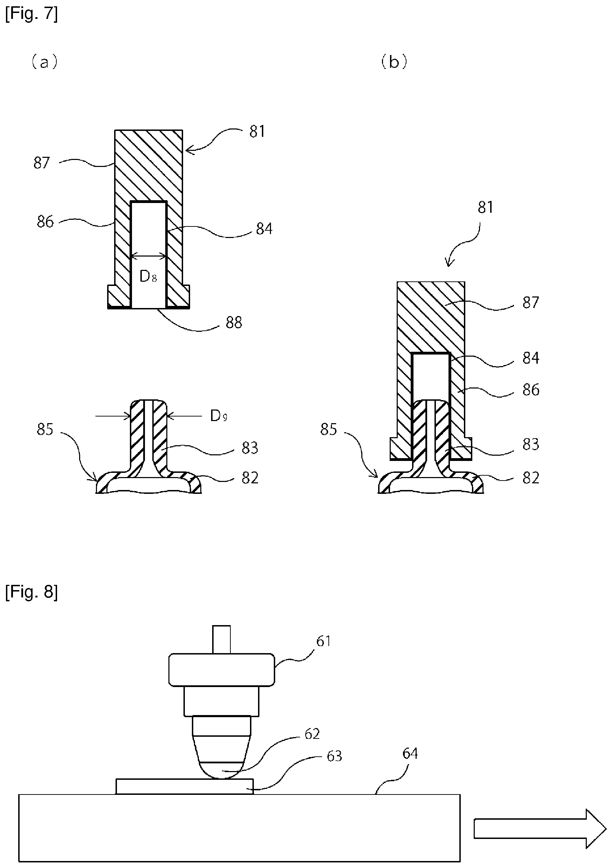

Fig. 7 (a) is a cross-sectional view showing an example of a nozzle cap for a medical syringe and a nozzle of a syringe barrel onto which the nozzle cap is fitted. Fig. 7 (b) is a cross-sectional view showing a state that the nozzle cap is fitted onto the nozzle. The nozzle cap 81 is integrally formed the medical rubber composition. The nozzle cap 81 comprises a cylindrical portion 86 that has an inner diameter D8 slightly smaller than an outer diameter D9 of the nozzle 83, and a needle-piercing portion 87 that is coupled to one end (the upper end in the figure) of the cylindrical portion 86. The needle-piercing portion 87 is formed into a cylindrical shape that has an outer surface continuous with the cylindrical portion 86. The other end (the lower end in the figure) of the cylindrical portion 86 is provided with an opening 88 for inserting the nozzle 83 into the cylindrical portion 86 to fit the nozzle cap 81 onto the nozzle 83. The inner surface of the nozzle cap 81 and the surface of the lower end of the cylindrical portion 86 are covered with an inert resin layer 84.

The outer surface of the nozzle cap 81 is not provided with an inert resin layer, and is irradiated with the ultraviolet ray. Since a friction coefficient and tackiness of the outer surface of the nozzle cap 81 is reduced, the problem that the nozzle caps stick to each other is improved.

## EXAMPLE

Next, the present invention will be described in detail by way of examples. However, the present invention is not limited to the examples described below. Various changes and modifications without departing from the spirit of the present invention are included in the scope of the present invention.

[Production of medical rubber article]

The materials shown in Table 1 were kneaded at a temperature of 60 °C for 20 minutes with an open roll to prepare medical rubber compositions. The obtained medical rubber compositions were crosslinked under a molding condition of 170 °C for 15 minutes, and then punched into circular slabs with a diameter of 28 mm and a thickness of 2 mm to form test pieces (the cured product of the medical rubber composition) for the ultraviolet ray irradiation. The test pieces for the ultraviolet ray irradiation were irradiated with the vacuum ultraviolet ray (wavelength: 172 nm) of the cumulative illuminance shown in Table 1.

The irradiation conditions of the ultraviolet ray were as follows.

·Irradiation apparatus: electrodeless excimer 172 nm irradiation apparatus (available from M.D.COM Inc.)
·Distance between cured product surface and lamp: 7 mm
·Arrival intensity of ultraviolet ray: 57.9 $mW/cm^2/sec$

Table 1

| | | Medical rubber article (composition) No. | 1 | 2 | 3 | 4 | 5 |
|---|---|---|---|---|---|---|---|
| Formulation (parts by mass) | (a) Base polymer | Chlorinated butyl rubber | 100 | 100 | 100 | 100 | - |
| | | Ordinary butyl rubber | - | - | - | - | 100 |
| | (b) Crosslinking agent | Triazine derivative | 1 | 1 | 1 | 1 | - |
| | | Sulfur | - | - | - | - | 0.5 |
| | | Zinc oxide | - | - | - | - | 3.0 |
| | Acid acceptor | Magnesium oxide | 3 | 3 | 3 | 3 | - |
| | Filler | Talc | - | 50 | 50 | 50 | 50 |
| | Vulcanization accelerator | Dithiocarbamic acid salt | - | - | - | - | 1.2 |
| | Pigment | Carbon black | 0.5 | 0.5 | 0.5 | 0.5 | 0.5 |
| | | Titanium oxide | 3 | 3 | 3 | 3 | 3 |
| | Oil | Process oil | 3 | 3 | 3 | 3 | 3 |
| Cumulative illuminance to cured product ($mJ/cm^2$) | | | 27370 | 17370 | 3474 | - | 17370 |
| Evaluation results | Friction coefficient | Static friction coefficient (μs) | 1.36 | 1.16 | 1.30 | 8.85 | 7.91 |
| | | Dynamic friction coefficient (μk) | 1.22 | 0.96 | 1.12 | 3.56 | 3.22 |
| | | Evaluation of static friction coefficient | G | G | G | P | P |
| | | Evaluation of dynamic friction coefficient | G | G | G | P | P |
| | Tackiness | Tackiness value (N) | 0.42 | 0.05 | 0.20 | 23.62 | 16.23 |
| | | Evaluation of tackiness | G | G | G | P | P |
| | Comprehensive evaluation | | G | G | G | P | P |

The details of the materials used are as follows.

Chlorinated butyl rubber: (Registered trademark) Chlorobutyl 1066 (chlorine amount: 1.25 wt%) available from Exxon Mobil Corporation Exxon
Ordinary butyl rubber: Exxon (Registered trademark) Butyl 268 (degree of unsaturation: 2.30 mole %) available from Exxon Mobil Corporation
Triazine derivative: ZISNET DB available from Sankyo Kasei Co., Ltd.
Sulfur: insoluble sulfur (SEIMI OT) available from Nippon Kanryu Industry Co., Ltd.
Zinc oxide: active zinc oxide AZO available from Seido Chemical Industry Co., Ltd.
Magnesium oxide: Magsarat 150s available from Kyowa Chemical Industry Co., Ltd.
Dithiocarbamic acid salt: Nocceler (Registered trademark) ZTC available from Ouchi Shinko Chemical Industrial Co., Ltd.

[Evaluation method]

(1) Friction coefficient

<Measurement of static friction coefficient and dynamic friction coefficient>

Fig. 8 is an illustrative drawing showing a method for measuring the friction coefficient of the medical rubber articles prepared above (the circular slabs with the thickness of 2 mm and the diameter of 28 mm irradiated with the ultraviolet ray) using a static and dynamic friction coefficient measuring machine TL201 (available from TAILAB Ltd.). The measuring sample 63 was fixed on a stage 64 on the lower side, and a 10-gram weight 61 was mounted on a dedicated probe provided with a SUS ball 62 having a diameter of 10 mm to allow the surface of the measuring sample 63 to contact with the SUS ball 62. Then, the stage 64 was moved in the direction of the arrow at a speed of 10 mm/second over a distance of 20 mm. A value obtained by dividing the friction force F generated during this process by the load (normal force) N was adopted as the friction coefficient $\mu$ ($\mu$=F/N). It is noted that the coefficient obtained by dividing the friction force F by the average normal force N1 over the moving distance of 20 mm was adopted as the dynamic friction coefficient, and the coefficient obtained by dividing the friction force F by the maximum average normal force N2 over the moving distance of 20 mm was adopted as the static friction coefficient.

<Evaluation of static friction coefficient>

The static friction coefficient was evaluated according to the following evaluation standard.
G: The static friction coefficient is less than 1.50.
F: The static friction coefficient is 1.50 or more and less than 3.3.
P: The static friction coefficient is 3.3 or more.
The static friction coefficient of less than 3.3 is accepted.

<Evaluation of dynamic friction coefficient>

The dynamic friction coefficient was evaluated according to the following evaluation standard.
G: The dynamic friction coefficient is less than 1.30.
F: The dynamic friction coefficient is 1.30 or more and less than 2.00.
P: The dynamic friction coefficient is 2.00 or more.
The dynamic friction coefficient of less than 2.00 is accepted.

(2) Tackiness test

<Measurement of tackiness>

The medical rubber articles prepared above (the circular slabs with the thickness of 2 mm and the diameter of 28 mm irradiated with the ultraviolet ray) were tested using a testing machine EZ-SX (available from Shimadzu Corporation). The medical rubber article was fixed on a dedicated jig on the lower side, and a SUS metal probe with $\Phi$10 mm on the upper side was pressed against the surface of the medical rubber article. After the set pressure (10 N) was reached, the pressure was held for 10 seconds, and the probe was raised at a speed of 10 mm/second. The peak value of the adhesion force generated between the probe and the cured product of the rubber composition was defined as the tackiness value. The measurement was conducted for n=5, and the average value of n=3 (excluding the maximum and minimum values) was adopted as the tackiness value.

<Evaluation of tackiness>

The tackiness was evaluated according to the following evaluation standard.
G: The tackiness value is 0.55 N or less.
P: The tackiness value is more than 0.55 N.

(3) Comprehensive evaluation

G: The evaluation result of the friction coefficient and the evaluation result of the tackiness are G or F.
P: The evaluation result of the friction coefficient and the evaluation result of the tackiness are both P.
The measuring results and evaluation results of the friction coefficient and tackiness are shown in Table 1. It can be seen from Table 1 that the medical rubber article obtained by the production method according to the present invention has a surface with a reduced friction coefficient and reduced tackiness.

## Industrial Applicability

The surface modification method according to the present invention can provide a medical rubber article having a surface with a reduced friction coefficient and reduced tackiness. The medical rubber article according to the present invention can be preferably used as a medical rubber article that is required to have a low friction coefficient or low tackiness (particularly a good sliding property).

The preferable embodiment (1) according to the present invention is a method for producing a medical rubber article, comprising a step of irradiating a cured product of a medical rubber composition containing a halogenated butyl rubber as (a) a base polymer with an ultraviolet ray.

The preferable embodiment (2) according to the present invention is the method for producing the medical rubber article according to the embodiment (1), wherein the ultraviolet ray has a wavelength ranging from 160 nm to 380 nm.

The preferable embodiment (3) according to the present invention is the method for producing the medical rubber article according to the embodiment (1) or (2), wherein cumulative illuminance of the ultraviolet ray to the cured product ranges from 1000 $mJ/cm^2$ to 50000 $mJ/cm^2$.

The preferable embodiment (4) according to the present invention is the method for producing the medical rubber article according to any one of the embodiments (1) to (3), wherein the halogenated butyl rubber includes at least one member selected from the group consisting of a chlorinated butyl rubber, a brominated butyl rubber, and a bromide of a copolymer of isobutylene and p-methylstyrene.

The preferable embodiment (5) according to the present invention is the method for producing the medical rubber article according to any one of the embodiments (1) to (4), wherein (a) the base polymer consists of the halogenated butyl rubber.

The preferable embodiment (6) according to the present invention is the method for producing the medical rubber article according to any one of the embodiments (1) to (5), wherein at least one part of a surface of the cured product is covered with an inert resin layer, and an exposed rubber surface that is not covered with an inert resin layer is irradiated with the ultraviolet ray.

The preferable embodiment (7) according to the present invention is the method for producing the medical rubber article according to the embodiment (6), wherein the inert resin layer is a layer made from a fluororesin.

The preferable embodiment (8) according to the present invention is the method for producing the medical rubber article according to the embodiment (6), wherein the inert resin layer is a layer made from a resin other than a fluororesin.

The preferable embodiment (9) according to the present invention is the method for producing the medical rubber article according to any one of the embodiments (1) to (8), wherein the medical rubber article is a rubber plug for a vial, a cap for a syringe, a plunger stopper, or a rubber plug for a vacuum blood collection tube.

The preferable embodiment (10) according to the present invention is the method for producing the medical rubber article according to the embodiment (6), wherein the medical rubber article is a plunger stopper for a syringe, having a liquid-contacting surface facing a liquid medication and a sliding surface contacting with a barrel, wherein the liquid-contacting surface is covered with an inert resin layer, and the sliding surface is not covered with an inert resin layer, thereby exposing the cured product of the medical rubber composition.

The preferable embodiment (11) according to the present invention is the method for producing the medical rubber article according to the embodiment (10), wherein the inert resin layer is a layer made from a fluororesin.

The preferable embodiment (12) according to the present invention is the method for producing the medical rubber article according to the embodiment (10), wherein the inert resin layer is a layer made from a resin other than a fluororesin.

## Claims

1. A method for producing a medical rubber article, comprising a step of irradiating a cured product of a medical rubber composition containing a halogenated butyl rubber as (a) a base polymer with an ultraviolet ray.

2. The method for producing the medical rubber article according to claim 1, wherein the ultraviolet ray has a wavelength ranging from 160 nm to 380 nm.

3. The method for producing the medical rubber article according to claim 1, wherein cumulative illuminance of the ultraviolet ray to the cured product ranges from 1000 $mJ/cm^2$ to 50000 $mJ/cm^2$.

4. The method for producing the medical rubber article according to claim 1, wherein the halogenated butyl rubber includes at least one member selected from the group consisting of a chlorinated butyl rubber, a brominated butyl rubber, and a bromide of a copolymer of isobutylene and p-methylstyrene.

**5.** The method for producing the medical rubber article according to claim 1, wherein (a) the base polymer consists of the halogenated butyl rubber.

**6.** The method for producing the medical rubber article according to claim 1, wherein at least one part of a surface of the cured product is covered with an inert resin layer, and an exposed rubber surface that is not covered with an inert resin layer is irradiated with the ultraviolet ray.

**7.** The method for producing the medical rubber article according to claim 6, wherein the inert resin layer is a layer made from a fluororesin.

**8.** The method for producing the medical rubber article according to claim 6, wherein the inert resin layer is a layer made from a resin other than a fluororesin.

**9.** The method for producing the medical rubber article according to any one of claims 1 to 8, wherein the medical rubber article is a rubber plug for a vial, a cap for a syringe, a plunger stopper, or a rubber plug for a vacuum blood collection tube.

**10.** The method for producing the medical rubber article according to claim 6, wherein the medical rubber article is a plunger stopper for a syringe, having a liquid-contacting surface facing a liquid medication and a sliding surface contacting with a barrel, wherein the liquid-contacting surface is covered with an inert resin layer, and the sliding surface is not covered with an inert resin layer, thereby exposing the cured product of the medical rubber composition.

**11.** The method for producing the medical rubber article according to claim 10, wherein the inert resin layer is a layer made from a fluororesin.

**12.** The method for producing the medical rubber article according to claim 10, wherein the inert resin layer is a layer made from a resin other than a fluororesin.

[Fig. 1]

30
31
32
38
35
33

[Fig. 2]

D1
47
40
42
H1
43
41
46
H2
44
H0
H3
45
48
49

[Fig. 3]

(a)
50
A
57
57
57
53a
53b
53
57
A
(b)
50
53
57
57
53a 53b
59
55
55

[Fig. 4]

(a)
50
B
57
57
B
53a
53b
53
57
(b)
50
53
57
53a 53b
59
55
55

[Fig. 5]

90
93
91

[Fig. 6]

(a)
93
96
94
97
(b)
93
96
94
97
95

[Fig. 7]

(a)
(b)

81
87
86
84
D8
88
D9
85
83
82
81
87
84
86
85
83
82

[Fig. 8]

61
62
63
64

**INTERNATIONAL SEARCH REPORT**

International application No. **PCT/JP2024/041244**

**A. CLASSIFICATION OF SUBJECT MATTER**

***C08J 7/00***(2006.01)i; ***A61J 1/06***(2006.01)i; ***A61M 5/315***(2006.01)i; ***A61M 5/32***(2006.01)i
FI: C08J7/00 304; A61J1/06 H; A61M5/315 512; A61M5/32 500

According to International Patent Classification (IPC) or to both national classification and IPC

**B. FIELDS SEARCHED**

Minimum documentation searched (classification system followed by classification symbols)

C08J7/00-7/02; C08J7/12-7/18; A61J1/06; A61M5/315; A61M5/32

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2012/165525 A1 (SUMITOMO RUBBER INDUSTRIES, LTD.) 06 December 2012 (2012-12-06)<br>claims, paragraphs [0025], [0035], [0072]-[0074], [0080], examples 1-13 | 1-5, 9<br>6-8, 10-12 |
| X<br>A | JP 2015-59165 A (SUMITOMO RUBBER INDUSTRIES, LTD.) 30 March 2015 (2015-03-30)<br>claims, paragraphs [0020], [0036]-[0038], [0042], [0045]-[0046], examples 1-5 | 1-5, 9<br>6-8, 10-12 |
| Y | JP 52-50345 A (DAIKYO GOMU SEIKO KK) 22 April 1977 (1977-04-22)<br>claims, p. 1, lower left column, lines 9-10, p. 2, upper left column, lines 5-12, example tables B, C | 1-5, 9 |
| Y | JP 2019-505407 A (BRIDGESTONE CORPORATION) 28 February 2019 (2019-02-28)<br>claims, paragraphs [0027]-[0028] | 1-5, 9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 January 2025** | **18 February 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/041244

C. DOCUMENTS CONSIDERED TO BE RELEVANT

| Category* | Citation of document, with indication, where appropriate, of the relevant passages | Relevant to claim No. |
|---|---|---|
| Y | JP 10-179690 A (DAIKYO SEIKO KK) 07 July 1998 (1998-07-07) paragraphs [0006]-[0007] | 1-5, 9 |
| Y | JP 2002-515268 A (ABBOTT LABORATORIES) 28 May 2002 (2002-05-28) paragraph [0029] | 1-5, 9 |
| Y | JP 2019-22581 A (MITSUBISHI GAS CHEMICAL COMPANY, INC.) 14 February 2019 (2019-02-14) claims, paragraphs [0015], [0025], [0030] | 1-5, 9 |
| A | JP 8-112342 A (DAIKYO SEIKO KK) 07 May 1996 (1996-05-07) claims, paragraphs [0009], [0023], examples 4-8, 10-14, 20 | 1-12 |

Form PCT/ISA/210 (second sheet) (July 2022)

**INTERNATIONAL SEARCH REPORT**
**Information on patent family members**

International application No.
**PCT/JP2024/041244**

| Patent document cited in search report | Publication date (day/month/year) | Patent family member(s) | Publication date (day/month/year) |
|---|---|---|---|
| WO 2012/165525 A1 | 06 December 2012 | US 2014/0128493 A1<br>claims, paragraphs [0043], [0052], [0079]-[0082], [0089]-[0090], examples 1-13<br>EP 2716669 A1<br>CN 103582659 A | |
| JP 2015-59165 A | 30 March 2015 | (Family: none) | |
| JP 52-50345 A | 22 April 1977 | (Family: none) | |
| JP 2019-505407 A | 28 February 2019 | US 2019/0224933 A1<br>claims, paragraphs [0025]-[0026]<br>CN 108367520 A<br>IT UB20156094 A1 | |
| JP 10-179690 A | 07 July 1998 | EP 841374 A2<br>p. 2, lines 35-50<br>AT 285445 T<br>CA 2220327 A1 | |
| JP 2002-515268 A | 28 May 2002 | WO 1999/055402 A1<br>p. 10, lines 12-19<br>CA 2329707 A | |
| JP 2019-22581 A | 14 February 2019 | (Family: none) | |
| JP 8-112342 A | 07 May 1996 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H03128941 A
- JP 2023053773 A